(19) Europäisches Patentamt

European Patent Office

Office européen des brevets

(11) Publication number: **0 359 206 B1**

(12) **EUROPEAN PATENT SPECIFICATION**

(45) Date of publication of patent specification: **29.03.95** (51) Int. Cl.⁶: **A61B 5/00**

(21) Application number: **89116878.3**

(22) Date of filing: **12.09.89**

(54) **Liver function testing apparatus.**

(30) Priority: **14.09.88 JP 230790/88**

(43) Date of publication of application:
**21.03.90 Bulletin 90/12**

(45) Publication of the grant of the patent:
**29.03.95 Bulletin 95/13**

(84) Designated Contracting States:
**DE FR GB IT**

(56) References cited:
**EP-A- 0 276 477**
**EP-A- 0 298 122**
**EP-A- 0 316 812**
**US-A- 4 017 192**

(73) Proprietor: **SUMITOMO ELECTRIC INDUS-
TRIES, LTD.**
**5-33, Kitahama 4-chome,**
**Chuo-ku**
**Osaka-shi,**
**Osaka 541 (JP)**

(72) Inventor: **Kanda, Masahiko**
**Osaka Works of Sumitomo Electric Indus-
tries, Ltd.**
**1-3 Shimaya 1-chome**
**Konohana-ku**
**Osaka (JP)**

(74) Representative: **Herrmann-Trentepohl, Wer-
ner, Dipl.-Ing. et al**
**Patentanwälte Herrmann-Trentepohl,
Kirschner, Grosse, Bockhorni & Partner
Forstenrieder Allee 59
D-81476 München (DE)**

EP 0 359 206 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

BACKGROUND OF THE INVENTION

Field of the Invention

The present invention relates to a liver function testing apparatus. More specifically, it relates to a liver function testing apparatus for automatically perf orming measurement for testing/diagnosing liver function by injecting a specific dye, which is selectively taken in and removed by only the liver, into blood and measuring a blood plasma disappearance rate and a retention rate thereof.

Description of the Prior Art

In general, the blood plasma disappearance rate and the retention rate have been measured by a method of blood sampling through use of indocyanine green (hereinafter referred to as ICG) serving as a specific dye.

According to this method, a tester intravenously injects ICG into an elbow vein, for example, of a testee with an injector to perform blood sampling three times after lapses of 5, 10 and 15 minutes from the injection, and separates blood serum upon coagulation of blood clot to measure absorbance at a wavelength of 805 nm through a spectrophotometer and obtain ICG concentration values in the blood serum after the lapses of 5, 10 and 15 minutes from a previously obtained calibration curve (corresponding ICG concentration in blood vs. absorbance), thereby to calculate the blood plasma disappearance rate and the retention rate from changes of the concentration values. ICG is dissolved in a physiological salt solution or the like.

Japanese Patent Publication Gazette No. 58649/1985 has proposed a method of applying light through the body surface of an organism and measuring quantities of light of a wavelength having high ICG absorption sensitivity and that of a wavelength substantially having no such sensitivity, thereby to measure the blood plasma disappearance rate and the retention rate from time changes (dye disappearance curve) thereof without performing blood sampling.

However, although it is necessary to correctly measure the blood sampling times after injection in the conventional blood sampling method, the times have not been accurately measured in an actual test, while the operation for such measurement has been complicated. Further, the testee has been subjected to heavy mental and physical burdens by blood sampling. In an $R_{MAX}$ measuringmethod of evaluating the blood plasma disappearance rate by performing measurement several times with changes in ICG dosages, which method has been widely employed in recent years, blood sampling is performed ten or more times, to further increase the burdens on the testee.

In the aforementioned method of performing measurement without blood sampling, which is disclosed in Japanese Patent Publication Gazette No. 58649/1985 or Japanese Patent Laying-Open Gazette No. 162934/1986, the output of a sensor actually attached to an organism is fluctuated by influences such as blood flow disturbance caused by suppression on a blood vessel, vibration of the organism, which is the object of measurement, pulsation in the organism, changes of blood volume in the organism (the blood volume is changed by merely vertically moving an arm, for example) etc., and hence a correct dye disappearance curve cannot be obtained. Thus, the blood plasma disappearance rate and the retention rate obtained by the curve cannot be recognized as being correct.

Further, there is disclosed a method of measuring ICG concentration in blood by employing widths between peaks of changes in quantities of light beams of two wavelengths caused by pulse waves through an optical blood measuring apparatus described in Japanese Patent Laying-Open Gazette No. 128387/1975 or an oximeter described in Japanese Patent Laying-Open Gazette No. 53-88778/1978 as another method of performing measurement without blood sampling. However, such widths of changes in quantities of light cannot be correctly measured due to vibration of the organism etc., and it has been impossible to obtain a correct dye disappearance curve.

SUMMARY OF THE INVENTION

Accordingly, a principal object of the present invention is to provide a liver function testing apparatus which can remove artifacts such as blood flow disturbance, vibration of an organism, pulsation in the organism and changes of the blood volume in the organism caused in attachment of a sensor to the organism, to enable correct measurement.

Briefly stated, a sensor formed by light sources and a light receiving element is attached to a testee before injection of a specific dye, to measure values $\Delta logT_1$ and $\Delta logT_2$ corresponding to pulse wave signals obtained upon passage through a prescribed optical path in vital tissue n times. Then a value $\alpha_0$ is evaluated by two-variable statistical computation as to n $\Delta logT_1$ and n $\Delta logT_2$ on the basis of an operation expression of $logT_1 = \alpha_0 logT_2$, and in response to decision outputs of levels of respective light beams emitted from the light sources, $\Delta logT_1$ and $\Delta logT_2$ corresponding to the pulse wave signals are measured on the basis of intensity levels of first light and second light reflecting the vital tissue from injection to a prescribed time after the specific dye is injected. A value Cg corresponding to specific dye concentration in blood is operated from $\alpha_0$, $\Delta logT_1$ and $\Delta logT_2$, and a function of a simulation curve in time changes of the operation result is operated through least square fitting, to output operation results of a blood plasma disappearance rate K and a T-minute retention rate R % of the specific dye on the basis of the function.

According to the present invention, therefore, time management of a correct specific dye disappearance curve is enabled, whereby correct data can be obtained. Further, the blood plasma disappearance rate K and the T-minute retention rate R % can be obtained not from several samples prepared according to the conventional blood sampling method but from a large number of disappearance curve data, thereby to improve reliability of the data. In addition, the method of measurement can be further simplified as compared with the conventional testing method of obtaining the blood plasma disappearance rate K and the T-minute retention rate R % by performing measurement three times with changes of ICG dosages. Further, the problematic artifacts such as blood flow disturbance, vibration of an organism, pulsation in the organism and changes of the blood volume in the organism caused upon attachment of a sensor to the organism can be removed, to enable correct measurement. Thus, the present invention is effectively applicable to the overall field of measuring a dye in an organism with no invasion.

In a more preferred embodiment of the present invention, $\Delta logT_1$ and $\Delta logT_2$ are measured m times as operation values Cg(T) assuming that $\Delta logT_1$ and $\Delta logT_2$ represent values corresponding to pulse wave signals of intensity levels of first light and second light passing through a prescribed optical path in vital tissue, and a value $\alpha(t)$ is evaluated as to m x 2 by two-variable statistical computation of $\Delta logT_1 = \alpha(t) \cdot \Delta logT_2$, to obtain $Cg(t) = \beta(\alpha(t) - \alpha_0)$.

In the preferred embodiment, further, the function Cg of the operated simulation curve is:

$$Cg = A \cdot e^{Bt}$$

where
Cg: operation value
t: elapsed time (min.) after injection of specific dye
A, B: constants
The blood plasma disappearance rate K and the T-minute retention rate R % are obtained from:

$$K = -B$$
$$R \% = e^{Bt}$$

assuming that the elapsed time after injection, which characteristically expresses intake of the specific dye in the liver, is T minutes.

These and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a schematic block diagram showing an embodiment of the present invention;
Fig. 2 illustrates incident light applied to an organism and transmitted light;
Fig. 3 illustrates changes in quantity of light corresponding to a pulse wave;
Fig. 4 illustrates changes of $\Delta logT_1$ and $\Delta logT_2$ expressed on x and y coordinates;
Figs. 5 and 6 are flow charts for illustrating concrete operation of the embodiment of the present invention;
Fig. 7 is a waveform diagram showing voltages corresponding to pulse waves; and
Fig. 8 illustrates an exemplary ICG disappearance curve.

DESCRIPTION OF THE PREFERRED EMBODIMENT

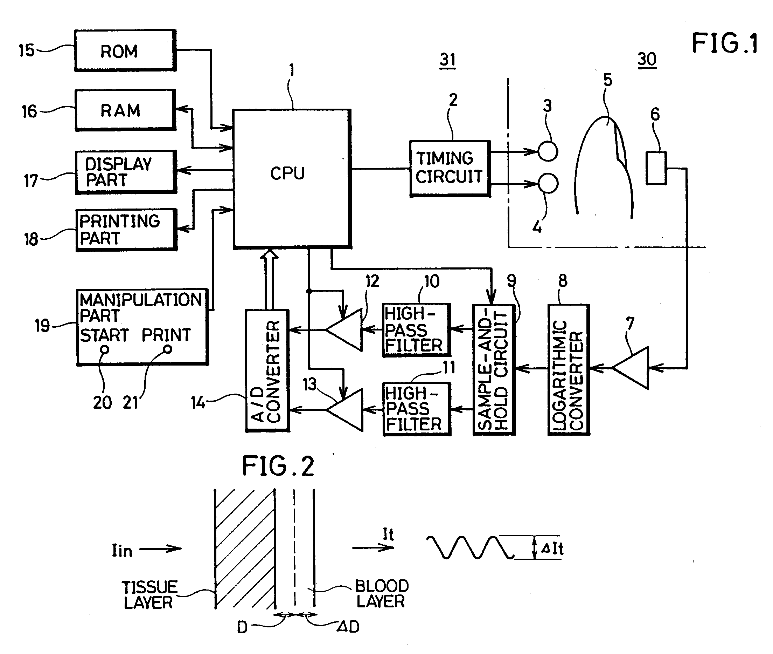

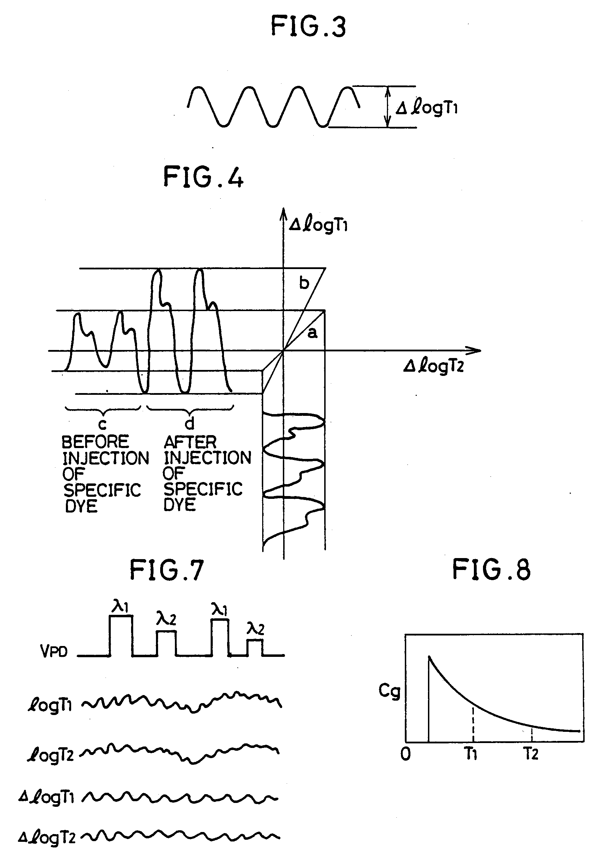

Fig. 2 illustrates incident light which is applied to vital tissue and transmitted light, Fig. 3 illustrates changes in quantity of light corresponding to a pulse wave, and Fig. 4 illustrates changes of $\Delta\log T_1$ and $\Delta\log T_2$ expressed on x and y coordinates.

With reference to Figs. 2 to 4, the principle of the present invention is now described. When incident light $I_{in}$ is applied to an organism as shown in Fig. 2, absorbance A is expressed as $\log I_t/I_{in}$, assuming that $I_t$ represents the quantity of transmitted light. The organism is formed by a tissue layer and a blood layer as shown in Fig. 2, and the blood layer is formed by an arterial layer and a venous layer. The thickness of the arterial layer is changed by $\Delta D$ in response to pulsation (pulse wave) of the heart. The quantity $I_t$ of the transmitted light is varied with this change. Therefore, the absorbance A is similarly changed by $\Delta A$. Hence,

$$\Delta A = \Delta\log I_t \qquad (1)$$

Assuming that $\Delta A_1$ and $\Delta A_2$ represent changes of absorption quantities caused by pulse waves of a wavelength $\lambda_1$ largely absorbed by a specific dye and an unabsorbed wavelength $\lambda_2$,

$$\Delta A_1 = (E_\beta^1 \cdot C_\beta + E_g^1 \cdot C_g) \cdot \Delta D \qquad (2)$$

$$\Delta A_2 = E_\beta^2 \cdot C_\beta \cdot \Delta D \qquad (3)$$

where
   $E_\beta^i$: absorption coefficient of blood at wavelength $\lambda_i$
   $E_g^i$: absorption coefficient of ICG at wavelength $\lambda_i$
   $C_\beta$: blood concentration
   $C_g$: specific dye concentration
   $\Delta D$: change in thickness of blood layer
   Assuming that the degree of oxygen saturation of the blood is constant,

$$E_\beta^1 \cdot C_\beta \cdot \Delta D = \alpha_0 (E_\beta^2 \cdot C_\beta \cdot \Delta D) \qquad (4)$$

Hence, the above expression (2) is transformed as follows:

$$\Delta A_1 = \alpha_0 \cdot \Delta A_2 + E_g^1 \cdot C_g \cdot \Delta D \qquad (5)$$

Thus,

$$C_g = (\Delta A_1/\Delta A_2 - \alpha_0) \cdot \frac{E_\beta^2 \cdot C_\beta}{E_g^1} \qquad \qquad \cdots (6)$$

$E_\beta^2/E_g^1$ is a known constant amount, and $C_\beta$ is interpreted as being constant as blood concentration. Further, $\alpha_0$ can be determined from relation between $\Delta A_{1,0}$ and $\Delta A_{2,0}$, since the equation (2) is expressed as:

$$\Delta A_{1,0} = E_\beta^1 \cdot C_\beta \cdot \Delta D \qquad (7)$$

before injection of the specific dye.

Hence, the specific dye concentration $C_g$ in the blood can be evaluated by obtaining $\Delta A_1/\Delta A_2$ after injection of the specific dye.

Assuming that $T_1$ and $T_2$ represent quantities of transmitted light having the wavelength $\lambda_1$ and that having the wavelength $\lambda_2$ while $\Delta T_1$ and $\Delta T_2$ represent changes thereof caused by $\Delta D$, the following equation results from the expression (1):

$$\Delta A_1/\Delta A_2 = \Delta\log T_1/\Delta\log T_2 = \alpha \qquad (8)$$

Hence, the expression (8) may be solved to obtain $\alpha$ before injection of the specific dye and the

4

EP 0 359 206 B1

expression (8) may be solved after injection of the specific dye, while Cg may be evaluated from the expression (6). In the oximeter described in the aforementioned Japanese Patent Laying-Open Gazette No. 53-88778/1978 etc., difference between peaks of changes in quantity of light corresponding to a pulse wave has been regarded as $\Delta\log T_1$, as shown in Fig. 3. However, this method can only prepare a sample corresponding to the cardiac cycle, and the above $\Delta\log T_1$ has been obtained by performing measurement several times and averaging the results in the actual circumstances.

According to the present invention, not the difference between the peaks is obtained but $\Delta\log T_1$ is set on the y-axis and $\Delta\log T_2$ is set on the x-axis as shown in Fig. 4, for example, so that changes of measured values are as shown in Fig. 4 respectively and move on the coordinates with inclination expressed by a straight line a before injection of the specific dye. This inclination is $\alpha_0$ shown in the expression (6). Then, when the specific dye is injected, absorbance of $\lambda_1$ is changed to define a waveform corresponding to a pulse wave such as d, and the inclination is changed to define a straight line such as b. This inclination $\alpha$ is $\Delta A_1/\Delta A_2$ in the expression (6).

Hence, inclination $\alpha(t)$ can be accurately calculated by increasing the number of measurement samples of $\Delta\log T_1$ and $\Delta\log T_2$, thereby to enable to detect of concentration changes of the specific dye at a high speed without depending on the cardiac cycle.

An embodiment according to this method is now described.

Fig. 1 is a schematic block diagram showing an embodiment of the present invention. Referring to Fig. 1, a liver function testing apparatus is formed by a sensor part 30 and a measurement processing part 31. The sensor part 30 includes a first light source 3, a second light source 4 and a light receiving element 6. The first light source 3 and the second light source 4 generate optical pulses of the wavelength $\lambda_1$ having large absorbance with respect to the specific dye and the wavelength $\lambda_2$ having no such absorbance. The light receiving element 6 receives light beams, which are applied from the light sources 3 and 4 to vital tissue 5 to pass through a prescribed optical path. The light sources 3 and 4 are controlled by a timing circuit 2 on the basis of a command from a CPU 1 which is provided in the measurement processing part 31, to alternately generate the light beams in pulse operation.

The CPU 1 included in the measurement processing part 31 serves as arithmetic means. As hereinabove described, the CPU 1 supplies prescribed pulses to the light sources 3 and 4 through the timing circuit 2. The light beams emitted from the first and second light sources 3 and 4 pass through the prescribed optical path in the vital tissue 5 to be incident upon the light receiving element 6. A current generated from the light receiving element 6 is subjected to current-voltage conversion and amplified by an amplifier 7. The amplified signal is supplied to a logarithmic converter 8 to be subjected to logarithmic conversion, and supplied to a sample-and-hold circuit 9, to be separated into signals of the wavelengths $\lambda_1$ and $\lambda_2$. The separated respective signals of the wavelengths $\lambda_1$ and $\lambda_2$ are supplied to high-pass filters 10 and 11. These signals include components by pulse waves as well as changes of blood volume such as those in venous blood, to have large snaking components. Therefore, the high-pass filters 10 and 11 remove these components to output only pulsating components, which in turn are supplied to an A-D converter 14 through amplifiers 12 and 13. The amplifiers 12 and 13 are so controlled that amplification factors thereof are changed in response to control signals from the CPU 1. The A-D converter 14 converts the inputted signals into digital signals and supplies the same to the CPU 1. The CPU 1 stores the digital signals in a RAM 16.

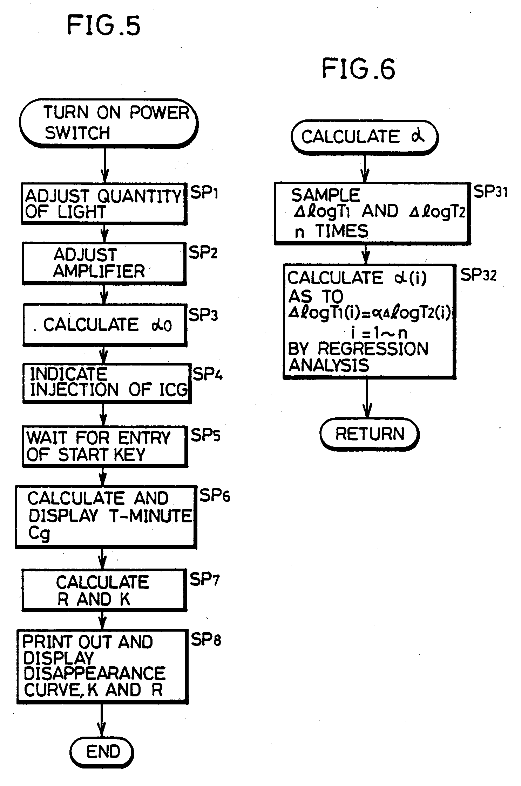

The CPU 1 is connected with a ROM 15, the RAM 16, a display part 17, a printing part 18 and a manipulation part 19. The ROM 15 stores programs based on flow charts shown in Figs. 5 and 6 as hereinafter described. The manipulation part 19 includes a start key 20 and a print key 21. The start key 20 is adapted to command starting of a measurement mode, and the print key 21 is adapted to supply a command for printing out test results to the printing part 18.

Figs. 5 and 6 are flow charts for illustrating concrete operation of the embodiment of the present invention, Fig. 7 is a waveform diagram showing voltages corresponding to pulse waves, and Fig. 8 illustrates an exemplary ICG disappearance curve obtained in the case of employing ICG as the specific dye.

With reference to Figs. 1 and 5 to 8, concrete operation of the embodiment of the present invention is now described as to the case of employing ICG as the specific dye. At a step SP1 shown in Fig. 5, power is applied to the apparatus and then quantities of light are adjusted. That is, the CPU 1 supplies a command to the timing circuit 2 to adjust driving currents for the light sources 3 and 4 respectively, while adjusting the light receiving element 6 so that its output reaches a prescribed level.

Light beams emitted from the light sources 3 and 4 pass through a prescribed optical path in the vital tissue 5 to be incident upon the light receiving element 6, and a current generated from the light receiving element 6 is subjected to current-voltage conversion and amplified by the amplifier 7, to provide an output

5

$V_{PD}$ shown in Fig. 7. This signal is supplied to the logarithmic converter 8 to be subjected to logarithmic conversion, and separated into signals of the waveforms $\lambda_1$ and $\lambda_2$ by the sample-and-hold circuit 9. These signals are expressed as $\log T_1$ and $\log T_2$ in Fig. 7 respectively. These signals contain components by pulse waves as well as changes in blood volume such as those in venous blood etc., to have large snaking components, which are removed by the high-pass filters 10 and 11 so that only pulsating components such as $\Delta \log T_1$ and $\Delta \log T_2$ shown in Fig. 7 are extracted.

At a step SP2, the CPU 1 controls amplification factors of the amplifiers 12 and 13 to amplify the signals until widths between peaks of pulse wave corresponding voltages of $\Delta \log T_1$ and $\Delta \log T_2$ shown in Fig. 7 reach certain levels. Then, the CPU 1 calculates $\alpha_0$ at a step SP3. In more concrete terms, the CPU 1 samples the signals of $\Delta \log T_1$ and $\Delta \log T_1$ n times at a step SP31 as shown in Fig. 6 and then performs regression analysis as to i = 1 to n through use of 2 x n data by an operation expression of $\log T_1(i) = \alpha \cdot \log T_2(i)$ at a step SP32 to calculate $\alpha$, and stores the same in the RAM 16 as $\alpha_0$.

Then, the CPU 1 displays indication such as "inject ICG", for example, on the display part 17 at a step SP4. Thus, the operator prepares to inject ICG into the vein of the organism, and turns on the start key 20 of the manipulation part 19 simultaneously with ICG injection. The CPU 1 waits for entry of the start key 20 at a step SP5, and operates T-minute ICG concentration Cg in blood at a step SP6 when the start key 20 is operated. In more concrete terms, $\alpha$ at a certain time t is evaluated in accordance with the aforementioned flow chart shown in Fig. 6, thereby to obtain Cg from the above expression (6), supposing that $\alpha$ is $\Delta A_1/\Delta A_2$. The data of Cg draw an ICG disappearance curve as shown in Fig. 8, for example, and within the data, constants A and B are evaluated through least square fitting with a simulation curve of:

$$Cg(l) = Ae^{Bt}$$
$$t = T_S/(n - 1) \text{ (min.)}$$

with respect to data between times $T_1$ and $T_2$ ($0 < T_1 < T_2 < T$).

Then, the CPU 1 operates a dye disappearance rate K = -B and a T-minute retention rate R % = $e^{Bt}$ at a step SP7, to evaluate K and R.

Then, the CPU 1 displays the disappearance curve shown in Fig. 8 and the values K and R on the display part 17, and outputs the same to the printing part 18 to print out the same at a step SP8.

The present invention can be also applied to an apparatus for measuring $R_{MAX}$ by evaluating/calculating values K of various ICG dosages.

According to the present invention, as hereinabove described, optical pulses of a wavelength largely absorbed by a specific dye and optical pulses of a wavelength not absorbed by the same are applied to vital tissue at prescribed levels to detect optical pulses passing through a prescribed optical path in the vital tissue, and after the specific dye is injected on the basis of the outputs, a dye disappearance rate and a retention rate of the specific dye are obtained on the basis of light receiving outputs from injection to a prescribed time in accordance with prescribed operation expressions. Thus, time management of a correct specific dye disappearance curve is enabled to obtain correct data.

Further, the dye disappearance rate and the retention rate can be obtained not from several samples prepared by the conventional blood sampling method but from a large number of disappearance curve data, thereby to improve reliability of the data.

In addition, the method of measurement can be further simplified as compared with the conventional testing method of obtaining the blood plasma disappearance rate and the retention rate by performing measurement several times with changes in ICG dosages.

Further, problematic artifacts such as blood flow disturbance, vibration of an organism, pulsation in the organism and changes of the blood volume in the organism caused in attachment of a sensor to the organism can be removed to enable correct measurement. Thus, the present invention is effectively applicable to the overall field of measuring a dye in an organism with no invasion.

The present invention is applicable not only to a liver function testing apparatus but to an apparatus, such as a pulse oximeter, for example, for measuring changes in concentration of a dye in an organism through pulse waves.

## Claims

1. A liver function testing apparatus for testing liver function, comprising:

   light source means (3,4) for exposing vital tissue to first light of a wavelength absorbed by a specific dye dosed into blood of said vital tissue to be taken in and removed by the liver and second light of a wavelength not absorbed by said specific dye;

EP 0 359 206 B1

light receiving means (6) for detecting said first light and said second light from said light source means passing through a prescribed optical path in said vital tissue;

decision means (SP1) for deciding levels of said first light and said second light from said light source means in response to signals received by said light receiving means;

set means (SP1) for setting levels of said first light and said second light emitted from said light source means so that intensity levels of said first light and said second light passing through said prescribed optical path in said vital tissue upon attachement of a sensor formed by said light source means and said light receiving means to a testee are within prescribed ranges according to the optical properties of the blood of the vital tissue, when the blood is free of said specific dye;

set means (SP2) for logarithmically converting intensitiy values $T_1$ and $T_2$ of said first light and said second light passing through said prescribed optical path in said vital tissue extracting only $\Delta logT_1$ and $\Delta logT_2$ corresponding to blood pulse wave signals, and setting maximum and minimum widths of said $\Delta logT_1$ and $\Delta logT_2$ within prescribed ranges according to the optical properties of the blood of the vital tissue when the blood is free of said specific dye; characterized by

means (SP4) for informing timing for injecting said specific dye;

arithmetic means (SP3 to SP8) for calculating n times $\Delta logT_1$ and $\Delta logT_2$ corresponding to blood pulse wave signals obtained by passage of the first and the second light through said prescribed optical path in said vital tissue upon attachment of said sensor formed by said light source means and said light receiving means to said testee to evaluate $\alpha0$ by statistical computation as to $\Delta logT_1$ and $\Delta logT_2$ on the basis of an operation expression of $\Delta logT_1 = \alpha_0 \Delta logT_2$ according to a state when the blood is free of said specific dye, measuring $\Delta logT_1$ and $\Delta logT_2$ n times corresponding to said blood pulse wave signals on the basis of intensity levels of said first light and second light which have been transmitted by said vital tissue from injection to a prescribed time, t, in response to outputs from said decision means in a state, when the blood contains said specific dye to give $\alpha = \Delta logT_1 / \Delta logT_2$, calculating a value Cg(t) proportional to $(\alpha - \alpha_0)$ corresponding to specific dye concentration in blood from said $\alpha_0$, and $\alpha$, fitting the calculated values of Cg(t) to a simulation function versus time by means of least square fitting and evaluating a blood plasma disappearance rate of said specific dye K and a T-minute retention rate R % of said specific dye on the basis of said function; and

output means (17, 18) for outputting the result of operation by said arithmetic means.

2. A liver function testing apparatus in accordance with claim 1, wherein said arithmetic means includes means for calculating $\Delta logT_1$ and $\Delta logT_2$ m times as operation values Cg(t) assuming that $\Delta logT_1$ and $\Delta logT_2$ represent values corresponding to blood pulse wave signals expressing intensity levels of said first light and said second light passing through said prescribed optical path in said vital tissue, evaluating $\alpha(t)$ as to m x 2 by two-variable statistical computation of $\Delta logT_1 = \alpha(t) \cdot \Delta logT_2$, and obtaining:

$$Cg(t) = \beta(\alpha(t) - \alpha_0)$$

where $\beta$ represents a constant.

3. A liver function testing apparatus in accordance with claim 1, wherein a function Cg of said simulation curve operated by said arithmetic means is:

$$Cg = A \cdot e^{Bt}$$

where

Cg: operation value

t: elapsed time (min.) after injection of specific dye

A, B: constants

said apparatus further including means for obtaining said blood plasma disappearance rate K and said T-minute retention rate R % from:

$$K = -B$$
$$R \% = e^{Bt}$$

assuming that an elapsed time upon injection, which characteristically expresses intake of said specific dye in the liver, is T minutes.

7

**Patentansprüche**

1. Vorrichtung zur Untersuchung der Leberfunktion umfassend:
   Lichtquelleneinrichtungen (3, 4), um ein lebendes Gewebe einem ersten Licht einer Wellenlänge, die von einem bestimmten Farbstoff absorbiert wird, welcher in das Blut des lebenden Gewebes eingegeben wird und von der Leber eingenommen und entfernt wird, und einem zweiten Licht einer Wellenlänge, die nicht von dem bestimmten Farbstoff absorbiert wird, auszusetzen;
   eine Lichtempfangseinrichtung (6) zum Detektieren des ersten Lichtes und des zweiten Lichtes von der Lichtquelleneinrichtung, das entlang eines vorgeschriebenen optischen Weg durch das lebende Gewebe hindurchtritt;
   eine Bestimmungseinrichtung (SP1) zum Bestimmen der Pegel des ersten Lichtes und des zweiten Lichtes von der ersten Lichtquelleneinrichtung in Antwort auf Signale, die von der Lichtempfangseinrichtung empfangen werden;
   eine Einstelleinrichtung (SP1) zum Einstellen der Pegel des ersten Lichtes und des zweiten Lichtes, die von der ersten Lichtquelleneinrichtung emittiert werden, so daß die Intensitätspegel des ersten Lichtes und des zweiten Lichtes, die durch den vorgeschriebenen optischen Weg in dem lebenden Gewebe laufen, beim Anbringen eines Sensors, der aus der ersten Lichtquelleneinrichtung und der zweiten Lichtempfangseinrichtung gebildet wird, an eine Testperson innerhalb von vorgeschriebenen Bereichen liegt, entsprechend den optischen Eigenschaften des Blutes des lebenden Gewebes, wenn das Blut frei von dem Farbstoff ist;
   eine Einstelleinrichtung (SP2) zum logarhithmischen Konvertieren der Intensitätswerte $T_1$ und $T_2$ des ersten Lichtes und des zweiten Lichtes, die durch den vorgeschriebenen optischen Weg in dem lebenden Gewebe hindurchgehen, wobei nur $\Delta \log T_1$ und $\Delta \log T_2$ entsprechend den Blutpulswellensignalen extrahiert werden, und wobei maximale und minimale Breiten von $\Delta \log T_1$ und $\Delta \log T_2$ innerhalb der vorgeschriebenen Bereiche entsprechend den optischen Eigenschaften des Blutes des lebenden Gewebes, wenn das lebende Gewebe frei ist von dem bestimmten Farbstoff, eingestellt werden;
   **gekennzeichnet durch**
   eine Einrichtung (SP4) zum Informieren der Zeit zum Injizieren des bestimmten Farbstoffes;
   Arithmetikeinrichtungen (SP3 bis SP8) zum n-fachen Berechnen von $\Delta \log T_1$ und $\Delta \log T_2$ entsprechend den Blutpulswellensignalen, die beim Durchgang des ersten und zweiten Lichtes durch den vorgeschriebenen optischen Weg in dem lebenden Gewebe erhalten werden beim Anbringen des Sensors, welcher durch die erste Lichtquelleneinrichtung und die Lichtempfangseinrichtung gebildet wird, an der Testperson, um $\alpha_0$ durch statistische Berechnung von $\Delta \log T_1$ und $\Delta \log T_2$ auf der Basis eines Berechnungsausdruckes $\Delta \log T_1 = \alpha_0 \Delta \log T_2$ zu berechnen, entsprechend einem Zustand, wenn das Blut frei von dem Farbstoff ist, zum n-fach Messen von $\Delta \log T_1$ und $\Delta \log T_2$ entsprechend den Blutpulswellensignalen auf der Basis der Intensitätspegel des ersten Lichtes und des zweiten Lichtes, welches durch das lebende Gewebe hindurchlaufen, beginnend von der Injektion bis zu einer vorgegebenen Zeit t, in Antwort auf die Ausgänge von der Bestimmungseinrichtung in einen Zustand, wenn das Blut den bestimmten Farbstoff enthält, um $\alpha = \Delta \log T1 / \Delta \log T2$ zu ergeben, zum Berechnen eines Wertes Cg(t) proportional zu $(\alpha-\alpha0)$ entsprechend der Konzentration des bestimmten Farbstoffes in dem Blut aus $\alpha_0$ und $\alpha$, durch Anpassung der berechneten Werte von Cg(t) an eine zeitabhängige Simulationsfunktion mittels der Methode der kleinsten Quadrate und zum Ableiten einer Blutplasmaschwundrate des bestimmten Farbstoffes K und einer T-Minuten-Rückhalterate R% des bestimmten Farbstoffes auf der Basis dieser Funktion; und
   Ausgabeeinrichtungen (17, 18) zum Ausgeben des Rechenergebnisses von der Arithmetikeinrichtung.

2. Vorrichtung zur Untersuchung der Leberfunktion gemäß Anspruch 1, worin die Arithmetikeinrichtung eine Einrichtung zum m-fachen Berechnen von $\Delta \log T_1$ und $\Delta \log T_2$ als Rechenwerte Cg(t) unter der Annahme umfaßt, daß $\Delta \log T_1$ und $\Delta \log T_2$ Werte darstellen entsprechend den Blutpulswellensignalen, die die Intensitätspegel des ersten Lichtes und des zweiten Lichtes widergeben, die auf dem vorgeschriebenen optischen Weg in dem lebenden Gewebe hindurchgegangen sind, wodurch $\alpha(t)$ m x 2-fach berechnet wird durch eine statistische Zweivariablenberechnungen von $\Delta \log T_1 = \alpha(t) \Delta \log T_2$ und erhalten:

$$Cg(t) = \beta(\alpha(t) - \alpha_0)$$

wobei $\beta$ eine Konstante widergibt.

**EP 0 359 206 B1**

3. Vorrichtung zur Untersuchung der Leberfunktion gemäß Anspruch 1, worin die Funktion Cg der Simulationskurve, die von der Arithmetikeinrichtung berechnet wird, gegeben ist durch:

$$Cg = A \cdot e^{Bt}$$

wobei
    Cg: Berechnungswert
    t: Verstrichene Zeit (Minuten) nach Injektion des Farbstoffes
    A, B: Konstanten

und wobei die Vorrichtung weiterhin eine Einrichtung zum Ermitteln der Blutplasmaschwundrate K und der T-Minuten-Rückhalterate R% umfaßt aus:

$$K = -B$$
$$R\% = e^{Bt}$$

unter der Annahme, daß die seit der Injektion verstrichene Zeit, welche die Aufnahme des bestimmten Farbstoffes in der Leber charakteristisch ausdrückt, gleich T Minuten ist.

**Revendications**

1. Dispositif pour examiner le fonctionnement du foie pour examiner le fonctionnement du foie, comprenant :
   - des moyens de source de lumière (3, 4) pour exposer du tissu vital à une première lumière, d'une longueur d'onde absorbée par un colorant spécifique dosé dans le sang dudit tissu vital devant pénétrer dans et être éliminé par le foie, et à une seconde lumière, d'une longueur d'onde non absorbée par ledit colorant spécifique;
   - des moyens de réception de lumière (6) pour détecter ladite première lumière et ladite seconde lumière provenant desdits moyens de source de lumière, passant à travers un chemin optique prescrit dans ledit tissu vital;
   - des moyens de décision (SP1) pour décider des niveaux de ladite première lumière et de ladite seconde lumière provenant desdits moyens de source de lumière, en réponse à des signaux reçus par lesdits moyens de réception de lumière ;
   - des moyens de définition (SP1) pour définir les niveaux de ladite première lumière et de ladite seconde lumière émises par lesdits moyens de source de lumière, de telle sorte que les niveaux des intensités de ladite première lumière et de ladite seconde lumière passant à travers ledit chemin optique prescrit dans ledit tissu vital lors de l'attachement d'un capteur, formé par lesdits moyens de source de lumière et lesdits moyens de réception de lumière, à un sujet examiné sont dans des domaines prescrits selon les propriétés optiques du sang du tissu vital, quand le sang est exempt dudit colorant spécifique;
   - des moyens de définition (SP2) pour convertir de manière logarithmique les valeurs $T_1$ et $T_2$ des intensités de ladite première lumière et de ladite seconde lumière passant à travers ledit chemin optique prescrit dans ledit tissu vital, extraire seulement $\Delta logT_1$ et $\Delta logT_2$ correspondants à des signaux d'ondes impulsionnelles dans le sang, et définir les largeurs maximales et minimales de $\Delta logT_1$ et $\Delta logT_2$ dans des domaines prescrits selon les propriétés optiques du sang du tissu vital, quand le sang est exempt dudit colorant spécifique; caractérisé par
   - des moyens (SP4) pour informer du moment de l'injection dudit colorant spécifique;
   - des moyens arithmétiques (SP3 à SP8) pour calculer n fois $\Delta logT_1$ et $\Delta logT_2$ correspondants à des signaux d'ondes impulsionnelles dans le sang, obtenus par le passage de ladite première lumière et de ladite seconde lumière à travers ledit chemin optique prescrit dans ledit tissu vital lors de l'attachement d'un capteur formé par lesdits moyens de source de lumière et lesdits moyens de réception de lumière audit sujet examiné, pour évaluer $\alpha_0$ par un calcul statistique en fonction de $\Delta logT_1$ et $\Delta logT_2$ à partir de l'expression mathématique $\Delta logT_1 = \alpha_0.\Delta logT_2$ dans l'état où le sang est exempt dudit colorant spécifique; mesurer n fois $\Delta logT_1$ et $\Delta logT_2$ correspondants auxdits signaux d'ondes impulsionnelles dans le sang, à partir des niveaux des intensités de ladite première lumière et de ladite seconde lumière qui ont été transmises par ledit tissu vital depuis l'injection jusqu'à un temps t prescrit, en réponse aux sorties desdits moyens de décision dans un état où le sang contient ledit colorant spécifique, pour obtenir $\alpha = \Delta logT_1/\Delta logT_2$; calculer une valeur Cg(t) proportionnelle à ($\alpha - \alpha_0$), correspondant à une

concentration du colorant spécifique dans le sang, à partir desdites valeurs de $\alpha_0$ et de $\alpha$; ajuster les valeurs calculées de Cg(t) à une fonction de simulation par rapport au temps, au moyen de la méthode d'égalisation des moindres carrés; et évaluer une vitesse de disparition dudit colorant spécifique dans le plasma sanguin, K, et un taux R % de rétention dudit colorant spécifique en T minutes à partir de ladite fonction ; et
- des moyens se sortie (17, 18) pour sortir le résultat de l'opération par lesdits moyens arithmétiques.

2. Dispositif pour examiner le fonctionnement du foie selon la revendication 1, dans lequel lesdits moyens arithmétiques comportent des moyens pour calculer $\Delta logT_1$ et$\Delta logT_2$ m fois en tant que valeurs de calcul de Cg(t), en supposant que $\Delta logT_1$ et $\Delta logT_2$ représentent des valeurs correspondant à des signaux d'ondes impulsionnelles dans le sang exprimant les niveaux des intensités de ladite première lumière et de ladite seconde lumière passant à travers ledit chemin optique prescrit dans ledit tissu vital; évaluer $\alpha(t)$ en tant que m x 2 par calculs statistiques à deux variables de $\Delta logT_1 = \alpha(t).\Delta logT_2$; et obtenir :

$$Cg(t) = \beta[\alpha(t) - \alpha_0]$$

où $\beta$ représente une constante.

3. Dispositif pour examiner le fonctionnement du foie selon la revendication 1, dans lequel une fonction Cg de ladite courbe de simulation calculée par lesdits moyens arithmétiques est :

$$Cg = A.e^{Bt}$$

où

Cg :       valeur calculée,
t :       temps écoulé (min) après l'injection de colorant spécifique,
A, B :       constantes,
ledit dispositif comportant en outre des moyens pour obtenir ledit taux k de disparition dans le plasma sanguin et ledit taux R % de rétention en T minutes, a partir de :

$$K = -B,$$
$$R \% = e^{Bt},$$

en supposant que le temps écoulé après l'injection, qui exprime de façon caractéristique la pénétration dudit colorant spécifique dans le foie, est de T minutes.

**FIG.1**

30

31

- 5
- 6
- 7 — (amplifier)
- 8 LOGARITHMIC CONVERTER
- 9 SAMPLE-AND-HOLD CIRCUIT
- 10, 11 HIGH-PASS FILTER
- 12, 13 (amplifiers)
- 14 A/D CONVERTER
- 2 TIMING CIRCUIT
- 3, 4
- 1 CPU
- 15 ROM
- 16 RAM
- 17 DISPLAY PART
- 18 PRINTING PART
- 19 MANIPULATION PART
- 20 START
- 21 PRINT

**FIG.2**

Iin →
It →
$\Delta$It
BLOOD LAYER
$\Delta$D
D
TISSUE LAYER

## FIG.3

## FIG.4

c
BEFORE
INJECTION
OF
SPECIFIC
DYE

d
AFTER
INJECTION
OF
SPECIFIC
DYE

## FIG.7

## FIG.8

# FIG.5

```
┌─────────────────────┐
│  TURN ON POWER      │
│  SWITCH             │
└─────────────────────┘
          │
          ▼
┌─────────────────────┐ SP1
│ ADJUST QUANTITY     │
│ OF LIGHT            │
└─────────────────────┘
          │
          ▼
┌─────────────────────┐ SP2
│   ADJUST            │
│   AMPLIFIER         │
└─────────────────────┘
          │
          ▼
┌─────────────────────┐ SP3
│ . CALCULATE $\alpha_0$ │
└─────────────────────┘
          │
          ▼
┌─────────────────────┐ SP4
│ INDICATE            │
│ INJECTION OF ICG    │
└─────────────────────┘
          │
          ▼
┌─────────────────────┐ SP5
│ WAIT FOR ENTRY      │
│ OF START KEY        │
└─────────────────────┘
          │
          ▼
┌─────────────────────┐ SP6
│ CALCULATE AND       │
│ DISPLAY T-MINUTE    │
│ Cg                  │
└─────────────────────┘
          │
          ▼
┌─────────────────────┐ SP7
│   CALCULATE         │
│   R AND K           │
└─────────────────────┘
          │
          ▼
┌─────────────────────┐ SP8
│ PRINT OUT AND       │
│ DISPLAY             │
│ DISAPPEARANCE       │
│ CURVE, K AND R      │
└─────────────────────┘
          │
          ▼
      ┌───────┐
      │  END  │
      └───────┘
```

# FIG.6

```
┌─────────────────────┐
│  CALCULATE $\alpha$ │
└─────────────────────┘
          │
          ▼
┌─────────────────────┐ SP31
│ SAMPLE              │
│ $\Delta \log T_1$ AND $\Delta \log T_2$ │
│ n TIMES             │
└─────────────────────┘
          │
          ▼
┌─────────────────────┐ SP32
│ CALCULATE $\alpha(i)$ │
│ AS TO               │
│ $\Delta \log T_1(i) = \alpha \Delta \log T_2(i)$ │
│      $i = 1 \sim n$ │
│ BY REGRESSION       │
│ ANALYSIS            │
└─────────────────────┘
          │
          ▼
      ┌─────────┐
      │ RETURN  │
      └─────────┘
```

13